(19) Europäisches Patentamt
European Patent Office
Office européen des brevets

(11) Publication number: **0 291 247 B1**

# (12) EUROPEAN PATENT SPECIFICATION

(45) Date of publication of patent specification: **16.09.92**

(51) Int. Cl.5: **C07C 233/01**, C07C 323/00, A61K 31/16, A61K 7/40, A61K 31/165

(21) Application number: **88304127.9**

(22) Date of filing: **06.05.88**

## (54) Novel cinnamoylamide derivatives.

(30) Priority: **07.05.87 JP 109722/87**

(43) Date of publication of application:
**17.11.88 Bulletin 88/46**

(45) Publication of the grant of the patent:
**16.09.92 Bulletin 92/38**

(84) Designated Contracting States:
**AT BE CH DE ES FR GB GR IT LI LU NL SE**

(56) References cited:
**EP-A- 0 173 516**
**DE-A- 2 515 914**

**CHEMICAL ABSTRACTS, vol. 108, no. 5, 1 February 1988, page 590, column 1, abstract no. 37394f, Columbus, Ohio, USA; & JP-A-62 198 653**

**PATENT ABSTRACTS OF JAPAN, vol. 9, no. 267 (C-310) (1990), 24 October 1985; & JP-A-60 116 657**

(73) Proprietor: **ONO PHARMACEUTICAL CO., LTD.**
**No. 14, Doshomachi 2-chome Higashi-ku**
**Osaka-shi Osaka(JP)**

(72) Inventor: **Nakai, Hisao**
**13-8, Nishikammuri 2**
**Takatsuki-city Osaka(JP)**
Inventor: **Terashima, Hiroshi**
**20-17, Okutenjincho 2**
**Takatsuki-city Osaka(JP)**
Inventor: **Arai, Yoshinobu**
**8-505, Wakayamadai 1-5**
**Shimamotocho Mishima-gun Osaka(JP)**

(74) Representative: **Bentham, Stephen et al**
**J.A. Kemp & Co. 14 South Square Gray's Inn**
**London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 291 247 B1

## Description

This invention relates to novel cinnamoylamide derivatives, to processes for their preparation, pharmaceutical compositions containing them and their use.

Many theories have been expounded concerning the origin of androgenic alopecia based on, for example, (1) an imbalance of hormones, (2) genetic factors, (3) circulatory failure or (4) nutrition. It has also been suggested that testosterone (androgenic hormone) played an important role in the generation of hair.

The theory of Adachi et al (Biochem. Biophys. Res. Commun., 41, 884 (1970)) in which the relation between testosterone and androgenic alopecia is proved by biochemical experiments, is as follows:

i) first, testosterone biosynthesized in the testis is converted into dihydrotestosterone by 5α-reductase present in, inter alia, hair follicles and sebaceous glands on the head;

ii) the dihydrotestosterone causes a substantial reduction in the activities of adenyl cyclase;

iii) the level of cyclic-Amp in cells decreases; and

iv) a lowering of energy generation of hairs and limbus and suppression of protein synthesis is induced.

According to this theory it is thought that, as a result of the series of phenomena just described, hairs in the growing phase shift to the resting phase, the terminal hairs change to soft hairs, and androgenic alopecia results.

A report by H.V. Schweikert (J. Clin. Endocr., 38, 811 (1974)) supports this theory in that large quantities of metabolites produced by 5α-reductase such as dihydrotestosterone are present in hair follicles of androgenic alopecia patients in quantities greater than those in females or healthy males.

It has also been reported that dihydrotestosterone produced from testosterone by 5α-reductase also plays an important physiological role in the generation of acnes (acne, pimples etc.) in addition to androgenic alopecia. J.B. Hay et al (Br. J. Dermatol., 91, 123 (1974)) has reported from a comparative study of the affected skin of acne-patients and healthy skin that the metabolism of testosterone by 5α-reductase was enhanced in the parts affected by aggravated acne.

G. Sansone et al (see J. Invest. Dermatol., 56, 366 (1971)) found that the ability to synthesise dihydrotestosterone from testosterone increased from two to twenty times in the affected part of acne-patients compared to that in healthy man, and suggested that dihydrotestosterone generated by 5α-reductase is strongly related to the generation or aggravation of acne.

Dihydrotestosterone is also related to hypertrophy of the prostate. Cowan et al (J. Steroid Biochemistry, 11, 609 (1979)) reported that much dihydrotestosterone existed in the prostate of prostatic hypertrophy patients. It has been reported (See J. Clinical Endocrinol. and Metabolism, 56, 139 (1983)) that the activity of 5α-reductase in the prostate of prostatic hypertrophy patients is abnormally increased.

It has become clear from these reports that dihydrotestosterone plays an important role in the generation and development of prostatic hypertrophy.

Research has been carried out to discover compounds active as 5α-reductase inhibitors: the compounds have been mainly steroids or derivatives thereof.

Widespread investigations have been carried out in order to discover compounds which have a non-steroidal structure and possess inhibitory activity on 5α-reductase. We have filed a number of applications directed to compounds wherein cinnamic acid or benzoic acids form amides with anilines, [See Japanese Patent Kokai Nos. 60-97946, 60-116657, 60-142936, 60-142941, 60-146855, 61-126061 (equivalent to EP 0173516), 62-198652 and 62-198653].

For example, EP 0173516 and Japanese Patent Kokai No. 61-126061 describe a very wide range of amide compounds which possess inhibitory activity on 5α-reductase. The compounds most closely related to those of the present invention, and which are described as possessing antagonistic activity on leukotrienes, inhibitory activity on 5α-reductase, on phospholipase and on aldose reductase are defined by the general formula:

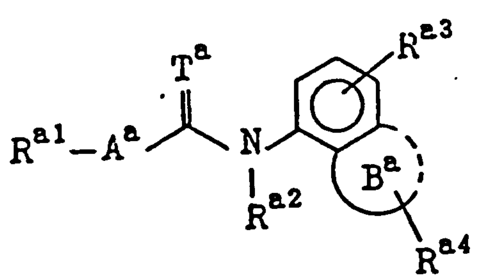

(A1)

[wherein $A^a$ represents a binylene group unsubstituted or substituted by one to three alkyl group(s) of from 1 to 10 carbon atom(s), $B^a$ represents a bivalent group $-O-CH_2-$ and $R^{a1}$ represents a group of the general formula:

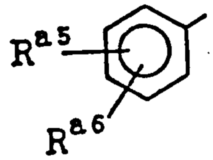

(wherein $R^{a5}$ and $R^{a6}$ represent, independently, a hydrogen atom or halogen atom or an alkyl, alkenyl or alkynyl group of from 1 to 20 carbon atom(s) in which from 1 to 5 carbon atoms may be replaced by an oxygen atom, sulfur atom, halogen atom, nitrogen atom, benzene ring, thiophene ring, naphthalene ring, carbon ring of from 4 to 7 carbon atoms, carbonyl group, carbonyloxy group, hydroxy group, carboxy group, azido group or nitro group),
$T^a$ represents an oxygen atom,
$R^{a2}$ represents a hydrogen atom,
$R^{a3}$ represents a hydrogen atom,
$R^{a4}$ represents a group $-(CH_2)_{ap}-COOR^{a8}$
(wherein ap represents an integer of from 1 to 10, $R^{a8}$ represents a hydrogen atom or an alkyl group of from 1 to 6 carbon atom(s)) ], i.e. the compounds of the general formula:

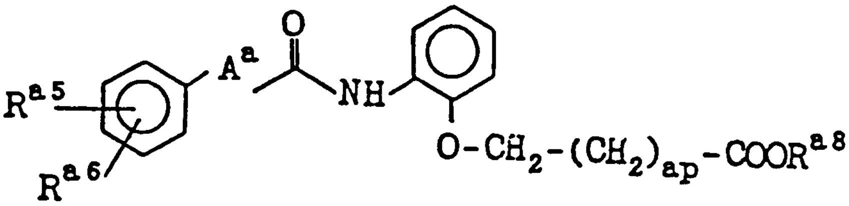

(A2)

(wherein all of the symbols are as hereinbefore defined).

Other compounds which are similar in chemical structure to the compounds of the present invention are disclosed in the specification of Japanese Patent Kokai No. 51-1438 and French Patent Publication No. 2164481.

In the specification of Japanese Patent Kokai No. 51-1438, it is disclosed that compounds including, inter alia, those defined by the general formula

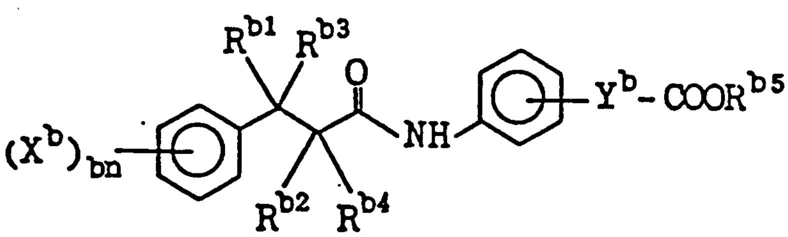

(B)

(wherein $R^{b1}$ and $R^{b2}$ each represent a hydrogen atom or lower alkyl group,
$R^{b3}$ represents a chemical bond together with $R^{b4}$,
$X^b$ represents a halogen atom, a lower alkyl group, a lower alkoxy group or a cyclic alkyl group,
bn represent an integer of from 1 to 3,
$R^{b5}$ represents a hydrogen atom and
$Y^b$ represent an oxyalkylene group bonded to the benzene ring via an oxygen atom) are active as antiallergic agents.

In the specification of French Patent Publication No. 2164481, it is disclosed that compounds including, inter alia, those of the general formula:

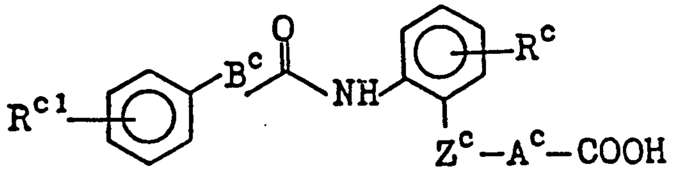

(C)

wherein $A^c$ represents an alkylene group of from 1 to 3 carbon atom(s), $B^c$ represent a bivalent ethylenic hydrocarbon group of from 1 to 5 carbon atom(s),
$R^c$ represents a hydrogen atom,
$R^{c1}$ represents one or two substituent(s) selected from alkyl, cycloalkyl, aryl, aralkyl, halogen, alkoxy, aryloxy and alkylthio groups, and
$Z^c$ represents an oxygen atom) possess anti-inflammatory and antipyretic action.

The compounds of the present invention described hereinafter, fall within the general scope of the formula designated above as (A2). However, none of the compounds has been previously prepared and all of them have been found to possess a surprisingly high level of inhibitory activity on 5α-reductase. There is no disclosure in EP 0173516 which might suggest that the compounds which have now been prepared might be of particular interest or that they might possess high activity in the inhibition of 5α-reductase.

The compounds of the present invention are not embraced by general formula (B), and the disclosure that the compounds of general formula (B) possess anti-inflammatory activity does not suggest that compounds of the same structure, or which are structurally related thereto, might possess inhibitory activity on 5α-reductase.

French Patent Publication No. 2164481 describes the synthesis of only a single compound. Moreover, the fact that compounds of the general formula (C) possesses anti-inflammatory activity does not suggest that such compounds, or related compounds, might possess inhibitory activity on 5α-reductase.

Accordingly, the present invention provides a cinnamoylamide derivative of the general formula:

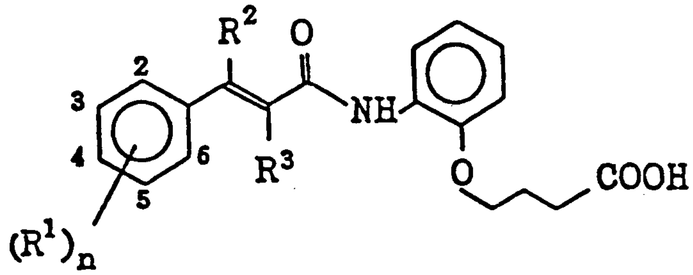

(I)

[wherein,
(i) when $R^2$ represent a methyl group and $R^3$ represents a hydrogen atom,
$(R^1)_n$ represents 3-pentyl, 4-pentyl, 4-neopentyl, 4-(2-ethylbutyl), 4-(2-methylpentyl), 2-fluoro-4-pentyloxy, 4-butylthio, 4-cyclobutylmethyl, 4-cyclohexylmethyl, 4-(4-phenylbutyl) or 4-phenoxy, and
(ii) when $R^2$ represents a hydrogen atom and $R^3$ represents a methyl group,
$(R^1)_n$ represents 3-pentyl or 4-phenethyl]
or a non-toxic salt thereof.

In the general formula (I), the configuration of the vinylene group to which $R^2$ and $R^3$ are bonded is E.

When $(R^1)_n$ represents a 4-(2-methylpentyl) group, two optical isomers arise owing to the asymmetric carbon atom in the pentyl group. The present invention includes these two isomers and mixtures thereof.

Preferred compounds of general formula (I) are those wherein, (i) when $R^2$ represents a methyl group and $R^3$ represent a hydrogen atom,
$(R^1)n$ represents 3-pentyl, 4-pentyl, 2-fluoro-4-pentyloxy, 4-cyclobutylmethyl, 4-cyclohexylmethyl or 4-phenoxy, and (ii) when $R^2$ represents a hydrogen atom and $R^3$ represents a methyl group,
$(R^1)_n$ represents 3-pentyl or 4-phenethyl.

The compounds wherein $R^2$ represents a methyl group, $R^3$ represent a hydrogen atom and $(R^1)_n$

represents 3-pentyl, 4-pentyl or 4-cyclobutylmethyl are especially preferred.

The compounds of general formula (I) may be converted into the corresponding salts by known methods. Water soluble salts are preferable. Suitable salts include, for example,

. salts of alkali metals, e.g. sodium and potassium,
. salts of alkaline earth metals, e.g. calcium and magnesium,
. ammonium salts,
. salts of pharmaceutically acceptable amines, e.g. tetraalkylammonium salts such as tetramethylammonium, triethylamine, methylamine, dimethylamine, cyclopentylamine, benzylamine, phenethylamine, piperidine, monoethanolamine, diethanolamine, tris (hydroxymethyl)-aminomethane, lysine, arginine and N-methyl-D-glucamine salts.

According to a feature of the present invention, the compounds of the general formula (I) may be prepared by saponifying the compounds of the general formula:

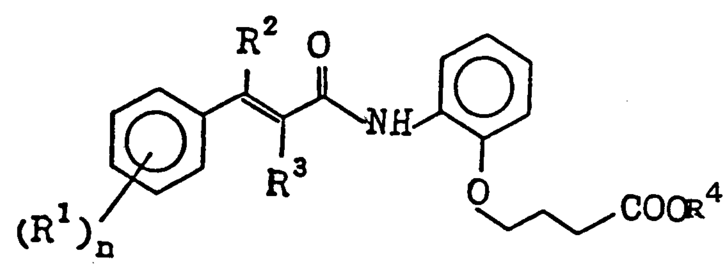

(II)

wherein $R^4$ represents a straight- or branched-chain alkyl group of 1 to 4 carbon atoms, preferably ethyl, and the other symbols are as hereinbefore defined.

The saponification may be carried out by known methods, for example, using an aqueous solution of alkali (e.g. potassium hydroxide, sodium hydroxide, lithium hydroxide, potassium carbonate or sodium carbonate) in a water-miscible organic solvent (e.g. tetrahydrofuran (THF), dioxan, ethanol or methanol). The reaction is generally carried out at a temperature of from -10 ° C to 100 ° C.

The compounds of the general formula (II) may be prepared by the reaction of a carboxylic acid of the general formula:

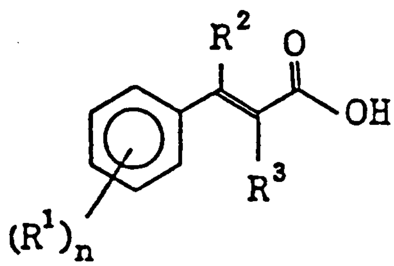

(III)

(wherein all of the symbols are as hereinbefore defined) or an activated derivative thereof with an amine of the general formula:

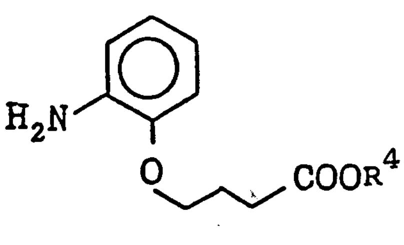

(IV)

wherein $R^4$ is as hereinbefore defined.

Methods for the preparation of an amide bond by reacting a carboxylic acid or an activated derivative thereof with an amine are known and include, for example,

(A) using a mixed acid anhydride as the activated derivative,

(B) using an acid halide as the activated derivative,

(C) using a condensing agent, e.g. dicyclohexylcarbodiimide (DCC).

Method (A) using a mixed acid anhydride may be carried out, for example, by reacting, a mixed acid anhydride obtained by acylating a carboxylic acid of the general formula (III) with an acyl halide (e.g. pivaloyl chloride, tosyl chloride or mesyl chloride) or an acid derivative (e.g. ethyl chloroformate or isobutyl chloroformate) in the presence of a tertiary amine (e.g. pyridine, triethylamine or picoline) in an inert organic solvent (e.g. chloroform, methylene chloride, diethyl ether or THF) or without solvent at from 0 ° C to 40 ° C, with an amine of the general formula (IV) in an inert organic solvent (for example one of those described above) at from 0 ° C to 40 ° C.

Method (B) using acid halide may be carried out, for example, by reacting, an acid halide obtained by treating a carboxylic acid of the general formula (III) with an acid halide (e.g. thionyl chloride or oxalyl chloride) in an inert organic solvent (e.g. as described above) at from -20 ° C to the reflux temperature of the solvent used, with an amine of the general formula (IV) in an inert organic solvent (e.g. as described above) at from 0 ° C to 40 ° C.

Method (C) using a condensing agent such as DCC may be carried out, for example, by reacting a carboxylic acid of the general formula (III) with an amine of the general formula (IV), using a condensing agent such as DCC, in the presence or absence of tertiary amine (e.g as described above), in an inert organic solvent (e.g as described above) or without solvent, at from 0 ° C to 40 ° C.

The reactions of (A), (B) and (C) are preferably carried out in an inert gas atmosphere (using e.g. argon or nitrogen) and under anhydrous conditions.

In the processes described in this specification, in each reaction, products may be purified by conventional methods, for example, distillation at atmospheric or reduced pressure, high performance liquid chromatography, thin layer chromatography using silica gel or magnesium silicate or washing or recrystallization. Purification may be carried out after each reaction or after a series of reactions.

Starting materials and reagents for use in the processes of the present invention are known or may be prepared by known methods.

For example, carboxylic acids of the general formula (III) may be prepared by the method described in the specification of Japanese Patent Kokai Nos. 60-97946, 60-116657, 60-142936, 60-142941 and 60-146855.

Amines of the formula (IV) may be prepared by the method described in the specification of Japanese Patent Kokai No. 61-126061.

The compounds of general formula (I) possess an inhibitory activity on 5α-reductase and are therefore useful for the prevention and/or treatment of diseases resulting from the excess generation of dihydrotestosterone in mammals, especially humans. Such diseases include, for example, alopecia, e.g. androgenic alopecia, acnes and hypertrophy of the prostate.

The high inhibitory activity on 5α-reductase of the compounds of the present invention is demonstrated by the screening system described hereafter.

<u>Inhibitory activity on 5α-reductase in vitro</u>

(1) Method

The test was carried out with reference to the method of J. Shimazaki et al [See Endocrinol., Japan., <u>18</u>, 179 (1971)].

Male rat's prostate (4g) was homogenized with three times its volume of 0.1M HEPES buffer (pH 7.4) including 0.25M cane sugar and was then centrifuged at 3000 r.p.m. for 10 mins.

The precipitate was suspended in the buffer solution described above (10ml), and the suspension was centrifuged at 3000 r.p.m. for 5 mins. The resulting precipitate was suspended in the buffer solution (3ml) described above and was used as a source of enzyme.

A reaction mixture (total volume 0.1ml) of [4-$C^{14}$]-testosterone (1.5n mol, 1.5 x $10^5$ cpm), NADPH (0.5 µmol), enzyme source (0.03ml) prepared as described above and several concentrations of the compounds of the present invention was incubated for 60 mins at 37 ° C. Enzyme reaction was quenched by addition of a mixture (0.4ml) of chloroform and methanol (1:2), and the mixture was centrifuged at 2000 r.p.m. for 3 mins. The supernatant (50 µl) was spotted on silica gel thin layer plate. The spot on the plate was developed with a mixture of chloroform, methanol and acetic acid (99.2 : 0.6 : 0.2). Radioactivity of the dihydrotestosterone on each plate was measured by radio-autography TLC scanning and an inhibitory ratio was calculated. The results are shown in the following Table 1.

Table 1: 5α-reductase inhibitory activity

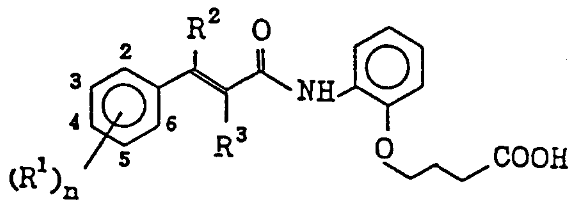

(I)

| Example No. | $(R^1)_n$ | $R^2$ | $R^3$ | 5α-reductase inhibitory activity $IC_{50}$ (μM) |
|---|---|---|---|---|
| 1 | $4-(CH_2)_4CH_3$ | $CH_3$ | H | 0.20 |
| 1 (a) | $3-(CH_2)_4CH_3$ | $CH_3$ | H | 0.16 |
| 1 (b) | $4-CH_2C(CH_3)_3$ | $CH_3$ | H | 0.37 |
| 1 (c) | $4-CH_2CH(CH_2CH_3)_2$ | $CH_3$ | H | 0.30 |
| 1 (d) | $4-CH_2CH(CH_3)(CH_2)_2CH_3$ | $CH_3$ | H | 0.34 |
| 1 (e) | $2-F-4-O(CH_2)_4CH_3$ | $CH_3$ | H | 0.28 |
| 1 (f) | $4-S(CH_2)_3CH_3$ | $CH_3$ | H | 0.43 |
| 1 (g) | $4-CH_2-$ 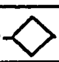 | $CH_3$ | H | 0.11 |
| 1 (h) | $4-CH_2-$ 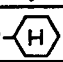 | $CH_3$ | H | 0.28 |
| 1 (i) | $4-(CH_2)_4-$ 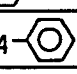 | $CH_3$ | H | 0.32 |
| 1 (j) | 4-O- 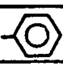 | $CH_3$ | H | 0.25 |
| 1 (k) | $3-(CH_2)_4CH_3$ | H | $CH_3$ | 0.24 |
| 1 (ℓ) | $4-(CH_2)_2-$ 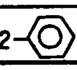 | H | $CH_3$ | 0.23 |
| Comparison: The 5α-reductase inhibitory activity of three compounds is given in EP 0173516 : the activities given are in the range shown. | | | | 2 - 5 |

(2) Results

The results of the test show that all of the compounds of the present invention possess a strong inhibitory activity on 5α-reductase. It will be seen that the inhibitory activities on 5α-reductase of the

compounds of the present invention are from 4.7 to 18.2 times as strong as the most active of the compounds for which 5α-reductase inhibitory activities are given in EP 0173516. The compounds of the present invention are consistently more active than the known compounds, the higher levels of activity found in the new compounds being completely unexpected from the prior art.

The high inhibitory activity on 5α-reductase of the compounds of the present invention render them useful for the prevention and/or treatment of disease resulting from excess generation of dihydrotestosterone in mammals, especially humans. Moreover, it was confirmed that the toxicity of the compounds of the present invention was very low so that they may be used as medicine with sufficient safety.

The compounds of the present invention will normally be administered systemically (mainly in the prevention and/or treatment of prostatic hypertrophy) or partially (mainly in the prevention and/or treatment of alopecia and acne), usually by oral or parenteral administration.

The doses to be administered are determined depending upon, for example, age, body weight, symptoms, the desired therapeutic effect, the route of administration, and the duration of the treatment. In the human adult, for the treatment and/or prevention of prostatic hypertrophy, the doses per person per dose are generally from 1 mg to 1 g, by oral administration, up to several times per day, and from 100 μg to 100 mg, by parenteral administration (preferably intravenous administration) up to several times per day.

In the human adult, for the treatment and/or prevention of alopecia and/or acne, the doses per person per dose are generally from 10 μg to 50 mg, by dermal administration up to several times per day.

As mentioned above, the doses to be used depend upon various factors. Therefore, there are cases in which doses lower than or greater than the ranges specified above may be used.

The present invention provides pharmaceutical compositions which comprise a compound of general formula I or a non-toxic salt thereof in association with a pharmaceutically acceptable carrier or coating.

The compounds of the present invention may be administered as solid compositions, liquid compositions or other compositions by oral administration and injections, external compositions and, e.g. suppositories, for parenteral administration.

Solid compositions for oral administration, include compressed tablets, pills, dispersible powders, capsules and granules. In such compositions, one or more of the active compound(s) is or are, admixed with at least one inert diluent (e.g. lactose, mannitol, glucose, hydroxypropylcellulose, microcrystalline cellulose, starch, polyvinylpyrrolidone or magnesium metasilicate aluminate). The compositions may also comprise, as is normal practice, additional substances other than inert diluents for example, lubricating agents (e.g. magnesium stearate), disintegrating agents (e.g. cellulose calcium gluconate), and agents to assist dissolution (e.g. glutamic acid or aspartic acid) and stabilizing agents (e.g. lactose).

The tablets or pills may, if desired, be coated with gastric or enteric material (e.g. sugar, gelatin, hydroxypropylcellulose or hydroxypropylmethylcellulose phthalate).

Capsules may be soft or hard.

Liquid compositions for oral administration include pharmaceutically-acceptable emulsions, solutions, suspensions, syrups and elixirs.

In such compositions, one or more of the active compound(s) is or are dissolved or dispersed in inert diluent(s) (e.g. purified water or ethanol).

Besides inert diluents, such compositions may also comprise adjuvants (e.g. wetting agents or suspending agents), sweetening agents, flavouring agents, perfuming agents and preserving agents.

Other compositions for oral administration include spray compositions which may be prepared by known methods and which comprise one or more of the active compound(s).

Spray compositions may comprise additional substances other than inert diluents, e.g. stabilizing agents (e.g. sodium sulfite), isotonic buffer (using, e.g. sodium chloride, sodium citrate, citric acid).

For the preparation of such spray compositions, for example, the method described in United States Patent No. 2868691 or 3095355 may be used.

Injectable compositions for parenteral administration include sterile aqueous or non-aqueous solutions, suspensions and emulsions. In such compositions, one or more of the active compound(s) is or are admixed with at least one inert aqueous diluent(s) (e.g. distilled water for injection or physiological salt solution) or inert non-aqueous diluent(s) [e.g. propylene glycol, polyethylene glycol, olive oil, ethanol, POLYSORBATE 80 (trade mark)]

Injectable compositions may comprise additional materials other than inert diluents e.g. preserving agents, wetting agents, emulsifying agents, dispersing agents, stabilizing agents (e.g. lactose), agents to assist dissolution (e.g. glutamic acid, aspartic acid).

They may be sterilized, for example, by filtration through a bacteria-retaining filter, by incorporation of sterilizing agents in the compositions or by irradiation. They may also be manufactured, for example, by

freeze drying, in the form of sterile solid compositions which can be dissolved in sterile water or some other sterile diluent for injection immediately before use.

Other compositions for parenteral administration include liquids for external use, and endermic liniments (e.g. ointments), suppositories and pessaries which comprise one or more of the active compound(s) and may be prepared by known methods.

Compositions for dermal administration, especially for the treatment and prevention of alopecia and acne, include liquids for external use such as lotions, tonics, sprays, solutions, suspensions, emulsions and liniments such as ointments, gels and creams.

Such compositions may comprise one or more active ingredient(s) and at least one inert diluent(s), for example, distilled water, a lower alcohol such as ethanol, a higher alcohol such as cetanol, a poly alcohol such as polyethylene glycol, propylene glycol, a cellulose derivative such as hydroxypropyl cellulose, animal or plant fats, vaseline, wax, silicone, a vegetable oil such as olive oil, a surfactant, or zinc oxide.

Besides inert diluents, such compositions may also comprise adjuvants (e.g. wetting agents, suspending agents, perfuming agents, preserving agents).

The following reference examples and examples illustrate the present invention.

The solvents in the parentheses show the developing or eluting solvents and the ratios of the solvents used are by volume in chromatographic separations.

Reference example 1

3-(4-pentylphenyl)-2EZ-butenoic acid ethyl ester

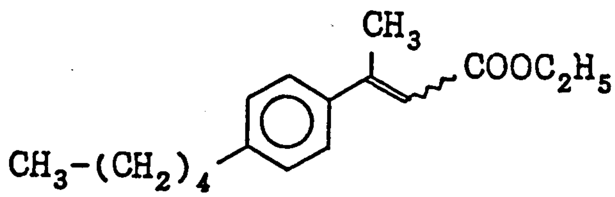

Sodium hydride (28.6g, content : 63%) was suspended in tetrahydrofuran (1000 ml). Diethyl ethoxycarbonylmethylphosphonate (168 g) dissolved in tetrahydrofuran (500 ml) was added dropwise into the mixture with cooling in an ice-bath over 30 mins. 4-Pentylacetophenone (95 g) in tetrahydrofuran (500 ml) was added to the solution. The solution was stirred overnight at a room temperature and then refluxed at 8 hrs. Tetrahydrofuran was evaporated from the reaction mixture. Diluted hydrochloric acid was added to the residue. The acidic solution was extracted with ethyl acetate. The extract was dried over magnesium sulfate and evaporated. The residue was purified by column chromatography on silica gel (n-hexane: ethyl acetate = 99 : 1 → 98 : 2 → 95 : 5) to give the title compound (128 g) having the following physical data:

TLC ; Rf 0.32 and 0.43 (n-hexane : ethyl acetate = 10 : 1).

Reference example 2

3-(4-pentylphenyl)-2E-butenoic acid

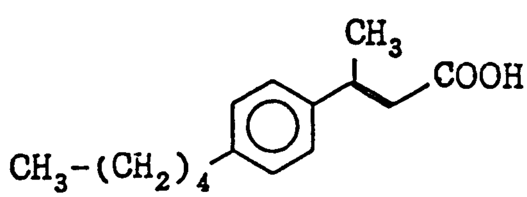

Ester (60.3g : prepared in reference example 1) was dissolved in a mixture of methanol (1300 ml) and tetrahydrofuran (800 ml). An aqueous solution of sodium hydroxide (2N ; 575 ml) was added to the solution. The solution was stirred at a room temperature for 1 hr and then at 50 ° C for 1 hr. The reaction mixture was extracted with ether to remove the neutral substance. The aqueous layer was acidified with 6N hydrochloric

acid. The acidic solution was dried over magnesium sulfate and then evaporated to give crude crystals. The crystals were recrystalized from n-hexane to give the title compound (32.7 g) having the following physical data:

TLC : Rf 0.40 (n-hexane : ethyl acetate = 2 : 1).

Reference example 3

4-(2-nitrophenoxy)butanoic acid ethyl ester

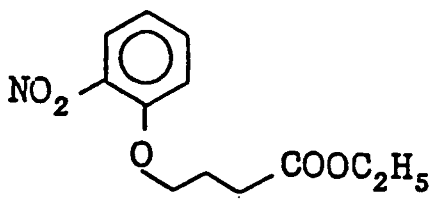

Sodium hydride (16.5 g, content : 62.4 %) was suspended in N,N-dimethylformamide (500 ml). 0-nitrophenol (6.0 g) dissolved in N,N-dimethylformamide (100 ml) was added dropwise into the mixture with stirring in ice-bath over about 20 mins. The mixture was stirred for 1 hr. at a room temperature.
4-bromobutanoic acid ethyl ester (84.2 g) dissolved in N,N-dimethylformamide (200 ml) was added to the mixture. The solution was stirred for 15 hrs at about 70 °C. N,N-dimethylformamide was evaporated in vacuo. Ethyl acetate (800 ml) was added to the residue. The mixture was washed with a water and a saturated brine, dried over magnesium sulfate and evaporated. The residue was purified by column chromatography on silica gel (n-hexane : ethyl acetate = 5 :1 → 3 : 1) to give the title compound (77.3g) having the following physical data :

TLC ; Rf 0.35 (n-hexane : ethyl acetate = 2 : 1).

Reference example 4

4-(2-aminophenoxy) butanoic acid ethyl ester

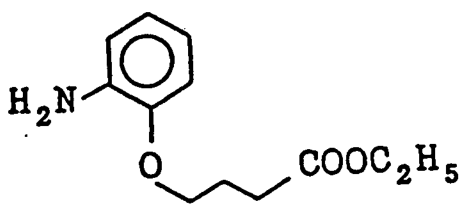

Nitro compound (77.0g; prepared in reference example 3) dissolved in ethanol (500 ml) was added to palladium carbon (13.1 g, content : 10%) suspended in a mixture of chloroform (100 ml) and ethanol (500 ml). The mixture was stirred for 10 hrs at a room temerature under an atmosphere of hydrogen. The reaction mixture was filtered to remove the catalyst. The filtrate was evaporated to give a white solid (79 g).

The obtained solid was dissolved in ethyl acetate (1000 ml). A saturated aqueous solution of sodium bicarbonate (500 ml) was added to the solution. The solution was stirred at a room temperature. The separated oily layer was washed with a saturated brine, dried over magnesium sulfate and evaporated. The residue was purified by column chromatography on silica gel (n-hexane : ethyl acetate : methylene chloride = 90 : 5 : 5→70 : 15 : 15) to give the title compound (60.0 g) having the following physical data:

TLC : Rf 0.43 (n-hexane : ethyl acetate : methylene chloride = 2 : 1 : 1).

Reference example 5

4-[2-(4-pentyl-β-methylcinnamoylamino)phenoxy]butanoic acid ethyl ester

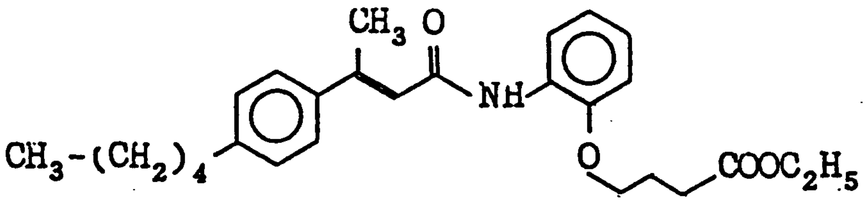

A mixture of butenoic acid derivative (58 g ; prepared in reference example 2) and oxalyl chloride (218 ml) was stirred for 1 hr at a room temperature. The obtained solution was evaporated to give the corresponding acid chloride. The acid chloride (obtained before) dissolved in methylene chloride (500 ml) was added dropwise into an amine (55.75 g ; prepared in reference example 4) dissolved in a mixture of methylene chloride (1500 ml) and pyridine (100 ml) in an ice-bath The mixture was stirred for 2 hrs at a room temperature. The reaction mixture was poured into diluted hydrochloric acid. The separated oily layer was washed with a diluted hydrochloric acid, a diluted aqueous solution of sodium hydroxide, water followed by a saturated brine, dried over sodium sulfate and then evaporated to give the title compound (114.5 g) having the following physical data as crude products. The product was used in the following reaction without purification.

TLC ; Rf 0.69 (n-hexane : ethyl acetate = 2 : 1).

Example 1

4-[2-(4-pentyl-β-methylcinnamoylamino)phenoxy]butanoic acid

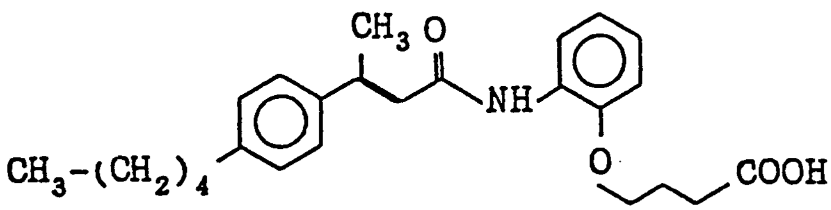

2N aqueous solution of sodium hydroxide (625 ml) was gradually added to the crude ethyl ester (114.5 g ; prepared in reference example 5) dissolved in a mixture of methanol (1500 ml) and tetrahydrofuran (500 ml). The mixture was stirred for 2 hrs at a room temperature. The reaction mixture was evaporated. 2N hydrochloric acid (650 ml) was added to the residue. The acidic solution was extracted with ethyl acetate. The extract was washed with water, followed by a saturated brine, dried over magnesium sulfate and evaporated to give crude crystals (pale yellow). The crystals were recrystallized from a mixture of n-hexane and benzene (2 : 3) to give the title compound (87 g) having the following physical data :

m.p. : 111 °C ;
TLC : Rf 0.54 (n-hexane : ethyl acetate = 1 : 2);
NMR : δ 8.45 (1H, m), 7.96 (1H, m), 7.41 (2H, d), 7.26 (2H, d), 6.96 (2H, m), 6.82 (1H, m), 6.28 (1H, m), 4.08 (2H, t), 2.60 (5H, m), 2.18 (2H, m), 1.61 (2H, m), 1.32 (4H, m), 0.90 (3H, t).

Hereinafter, using the corresponding acetophenone instead of 4-pentylacetophenone used in reference example 1, the compounds of the present invention shown in the following Table 2 were obtained by the same procedure as described in reference example 1 ~ 5 and example 1. Moreover, using the corresponding benzaldehyde instead of 4-pentylacetophenone used in reference example 1, or diethyl 1-ethoxycarbonyl-1-ethyl phosphonate instead of diethyl ethoxycarbonylmethyl phosphonate, the compounds of the present invention shown in the following Table 3 were obtained by the same procedure as described in reference example 1 ~ 5 and example 1.

Table 2:

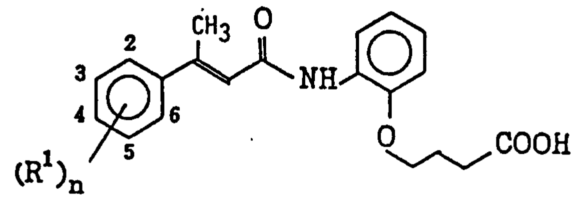

| Example No. | $(R^1)_n$ | Name | TLC | melting point or IR |
|---|---|---|---|---|
| 1 (a) | 3-$(CH_2)_4CH_3$ | 4-[2-(3-pentyl-β-methylcinnamoylamino)phenoxy] butanoic acid | Rf 0.23 (n-hexane: ethyl acetate =2:1) | 86 ~ 87°C |
| 1 (b) | 4-$CH_2C(CH_3)_3$ | 4-[2-(4-neopentyl-β-methylcinnamoylamino)phenoxy] butanoic acid | Rf 0.39 (n-hexane: ethyl acetate =1:1) | 130.5 ~ 132°C |
| 1 (c) | 4-$CH_2CH(CH_2CH_3)_2$ | 4-[2-{4-(2-ethylbutyl)-β-methylcinnamoylamino}phenoxy] butanoic acid | Rf 0.37 (n-hexane: ethyl acetate =2:1) | 102 ~ 104°C |
| 1 (d) | 4-$CH_2CH(CH_3)(CH_2)_2CH_3$ | 4-[2-{4-(2-methylpentyl)-β-methylcinnamoylamino}phenoxy] butanoic acid | Rf 0.55 (n-hexane: ethyl acetate =1:1) | 130.5 ~ 131°C |

Table 2 (continued)

| Example No. | $(R^1)_n$ | Name | TLC | melting point or IR |
|---|---|---|---|---|
| 1 (e) | 2-F & 4-$O(CH_2)_4CH_3$ | 4-[2-(2-fluoro-4-pentyloxy-β-methylcinnamoylamino)phenoxy] butanoic acid | Rf 0.29 (n-hexane: ethyl acetate =1:1) | 109 ~ 109.5°C |
| 1 (f) | 4-$S(CH_2)_3CH_3$ | 4-[2-(4-butylthio-β-methylcinnamoylamino)phenoxy] butanoic acid | Rf 0.28 (n-hexane: ethyl acetate =2:1) | 122°C |
| 1 (g) | 4-$CH_2$- 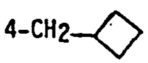 | 4-[2-(4-cyclobutylmethyl-β-methylcinnamoylamino)phenoxy] butanoic acid | Rf 0.40 (n-hexane: ethyl acetate =1:2) | 128 ~ 129°C |
| 1 (h) | 4-$CH_2$- 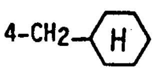 | 4-[2-(4-cyclohexylmethyl-β-methylcinnamoylamino)phenoxy] butanoic acid | Rf 0.35 (n-hexane: ethyl acetate =1:1) | 138.5 ~ 140°C |
| 1 (i) | 4-$(CH_2)_4$- 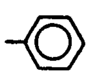 | 4-[2-{4-(4-phenylbutyl)-β-methylcinnamoylamino}phenoxy] butanoic acid | Rf 0.25 (n-hexane: ethyl acetate =2:1) | ν 3325, 3100~2300, 1710, 1640, 1610, 1530, 1450, 1255, 1180, 940, 740 $cm^{-1}$ |

Table 2 (continued)

| Example No. | $(R^1)_n$ | Name | TLC | melting point or IR |
|---|---|---|---|---|
| 1 (j) | 4-O- 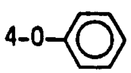 | 4-[2-(4-phenoxy-β-methylcinnamoylamino)phenoxy] butanoic acid | Rf 0.25 (n-hexane: ethyl acetate =1:1) | 141.5 ~ 143°C |

Table 3:

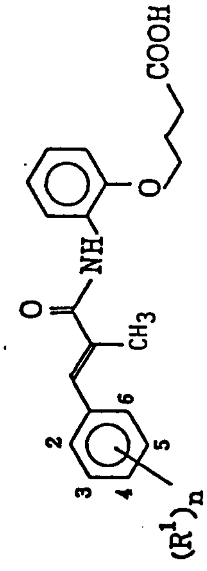

| Example No. | $(R^1)_n$ | Name | TLC | melting point or IR |
|---|---|---|---|---|
| 1 (k) | 3-$(CH_2)_4CH_3$ | 4-[2-(3-pentyl-α-methylcinnamoylamino)phenoxy] butanoic acid | Rf 0.22 (n-hexane: ethyl acetate =1:1) | ν 3470, 3300~2300, 1715, 1670, 1605, 1530, 1450, 750 cm-1 |
| 1 (ℓ) | 4-$(CH_2)_2$ – 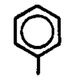 | 4-[2-(4-phenethyl-α-methylcinnamoylamino)phenoxy] butanoic acid | Rf 0.49 (ethyl acetate) | ν 3450, 3050~2800, 1718, 1670, 1600, 1525, 1450, 1290, 1260, 1220, 750 cm-1 |

Preparation example

Preparation of tablets containing 4-[2-(4-pentyl-β-methylcinnamoylamino)phenoxy]butanoic acid

4-[2-(4-pentyl-β-methylcinnamoylamino)phenoxy] butanoic acid (5 g), Cellulose calcium gluconate

(disintegrating agent : 200 mg), Magnesium stearate (lubricating agent : 100 mg) and Microcrystaline cellulose (4.7 g) were admixed in conventional method and punched out to obtain 100 tablets each containing 50 mg of active ingredient.

## Claims

**Claims for the following Contracting States : AT, BE, CH, DE, FR, GB, IT, LI, LU, NL, SE**

**1.** A cinnamoylamide derivative of the general formula:

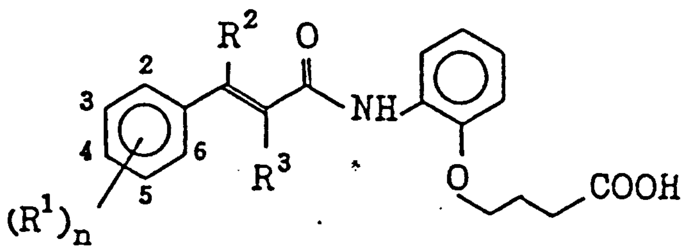

(I)

[wherein,
(i) when $R^2$ represents a methyl group and $R^3$ represents a hydrogen atom,
$(R^1)_n$ represents 3-pentyl, 4-pentyl, 4-neopentyl, 4-(2-ethylbutyl), 4-(2-methylpentyl), 2-fluoro-4-pentyloxy, 4-butylthio, 4-cyclobutylmethyl, 4-cyclohexylmethyl, 4-(4-phenylbutyl) or 4-phenoxy, and
(ii) when $R^2$ represents a hydrogen atom and $R^3$ represents a methyl group,
$(R^1)_n$ represents a group 3-pentyl or 4-phenethyl] or a non-toxic salt thereof.

**2.** A compound according to claim 1, wherein
(i) $R^2$ represents a methyl group and $R^3$ represents a hydrogen atom,
$(R^1)_n$ represents 3-pentyl, 4-pentyl, 2-fluoro-4-pentyloxy, 4-cyclobutylmethyl, 4-cyclohexylmethyl or 4-phenoxy, and
(ii) when $R^2$ represents a hydrogen atom and $R^3$ represents a methyl group,
$(R^1)_n$ represents 3-pentyl or 4-phenethyl.

**3.** A compound according to claim 2, wherein $R^2$ represents a methyl group, $R^3$ represents a hydrogen atom and $(R^1)_n$ represents 3-pentyl, 4-pentyl or 4-cyclobutylmethyl.

**4.** A process for the preparation of a cinnamoylamide derivative according to claim 1 which comprises saponifying a compound of the general formula:

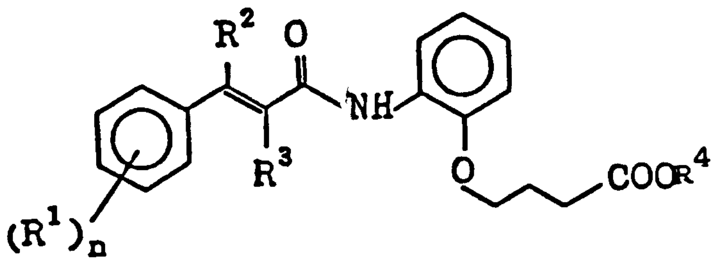

(II)

wherein $R^4$ represents a straight- or branched-chain alkyl group of 1 to 4 carbon atoms and the other symbols are as defined in claim 1, and optionally converting the compound of general formula (I) obtained into a non-toxic salt thereof.

**5.** A compound of general formula (II) depicted in claim 4 wherein $R^4$ is as defined in claim 4 and the other symbols are as defined in claim 1.

**6.** A process for the preparation of a compound according to claim 4 which comprises reacting a

compound of the general formula:

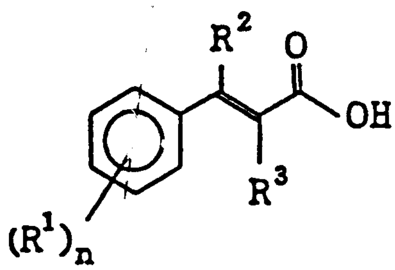

(III)

(wherein all of the symbols are as defined in claim 1) or an activated derivative thereof with an amine of the general formula:

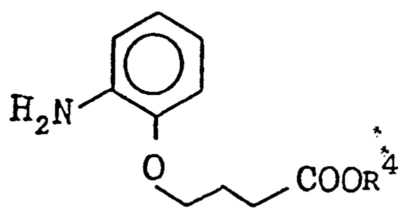

(IV)

wherein $R^4$ is as defined in claim 4.

7. A pharmaceutical composition which comprises a cinnamoylamide derivative according to claim 1 or a non-toxic salt thereof in association with a pharmaceutically acceptable carrier or coating.

8. A cinnamoylamide derivative according to claim 1 or a non-toxic salt thereof for use as a medicament.

9. Use of a cinnamoylamide derivative according to claim 1 or a non-toxic salt thereof in the manufacture of a medicament for the treatment of alopecia, acne or prostatic hypertrophy.

10. A cosmetic composition which comprises a cinnamoylamide derivative according to claim 1 or a non-toxic salt thereof.

11. Use as a cosmetic product in the treatment of acne or alopecia of a cinnamoylamide derivative according to claim 1 or a non-toxic salt thereof.

**Claims for the following Contracting States : ES, GR**

1. A process for the preparation of a cinnamoylamide derivative of the general formula:

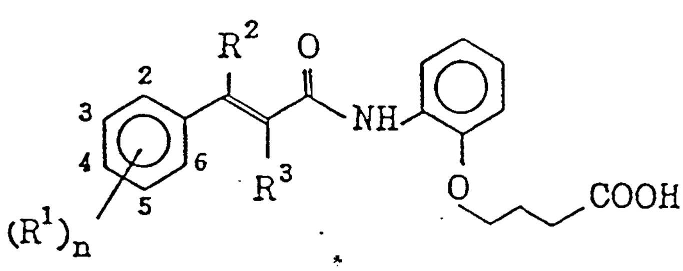

(I)

[wherein,

(i) when $R^2$ represents a methyl group and $R^3$ represents a hydrogen atom,

$(R^1)_n$ represents 3-pentyl, 4-pentyl, 4-neopentyl, 4-(2-ethylbutyl), 4-(2-methylpentyl), 2-fluoro-4-pentyloxy, 4-butylthio, 4-cyclobutylmethyl, 4-cyclohexylmethyl, 4-(4-phenylbutyl) or 4-phenoxy, and

(ii) when $R^2$ represents a hydrogen atom and $R^3$ represents a methyl group, $(R^1)_n$ represents a group 3-pentyl or 4-phenethyl] or a non-toxic salt thereof, which process comprises saponifying a compound of the general formula:

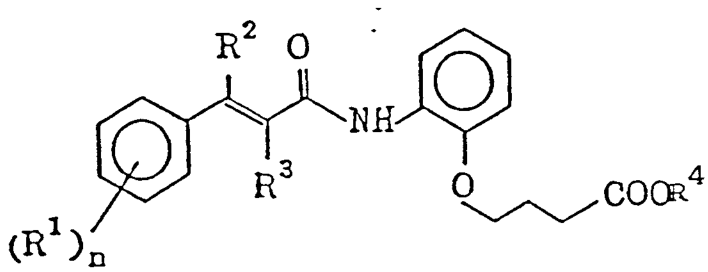

(II)

wherein $R^4$ represents a straight- or branched-chain alkyl group of 1 to 4 carbons atoms and the other symbols are as defined in relation to general formula (I) and optionally converting the compound of general formula (I) obtained in into a non-toxic salt thereof.

**2.** A process according to claim 1, for the preparation of a compound of formula (I) or a non-toxic salt thereof wherein (i) $R^2$ represents a methyl group and $R^3$ represents a hydrogen atom, $(R^1)_n$ represents 3-pentyl, 4-pentyl, 2-fluoro-4-pentyloxy, 4-cyclobutylmethyl, 4-cyclohexylmethyl or 4-phenoxy, and
(ii) when $R^2$ represents a hydrogen atom and $R^3$ represents a methyl group, $(R^1)_n$ represents 3-pentyl or 4-phenethyl.

**3.** A process according to claim 2 for the preparation of a compound of formula (I) or a non-toxic salt thereof wherein $R^2$ represents a methyl group, $R^3$ represents a hydrogen atom and $(R^1)_n$ represents 3-pentyl, 4-pentyl or 4-cyclobutylmethyl.

**4.** A process for the preparation of a compound of general formula (II) depicted in claim 1 which process comprises reacting a compound of the general formula:

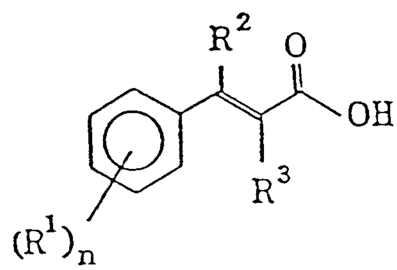

(III)

(wherein all of the symbols are as defined in claim 1) or an activated derivative thereof with an amine of the general formula:

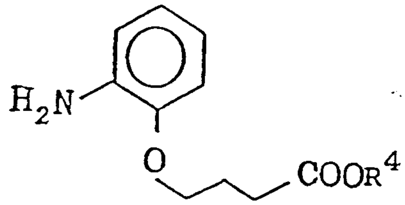

(IV)

wherein $R^4$ is as defined in claim 1.

5. A cosmetic composition which comprises a cinnamoylamide derivative of formula (I) as defined in claim 1 or a non-toxic salt thereof.

**Patentansprüche**

**Patentansprüche für folgende Vertragsstaaten : AT, BE, CH, DE, FR, GB, IT, LI, LU, NL, SE**

1. Cinnamoylamidderivat der allgemeinen Formel:

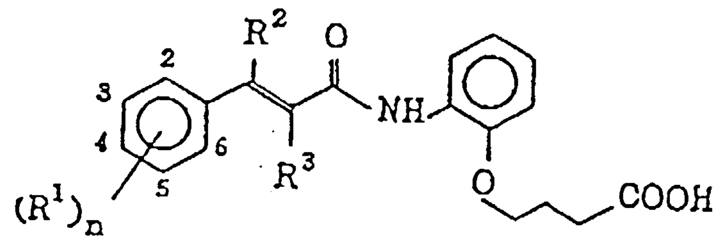

(I)

worin bedeuten:
(i) wenn $R^2$ für eine Methylgruppe steht und $R^3$ ein Wasserstoffatom darstellt,
$(R^1)_n$ 3-Pentyl, 4-Pentyl, 4-Neopentyl, 4-(2-Ethylbutyl), 4-(2-Methylpentyl), 2-Fluor-4-pentyloxy, 4-Butylthio, 4-Cyclobutylmethyl, 4-Cyclohexylmethyl, 4-(4-Phenylbutyl) oder 4-Phenoxy und
(ii) wenn $R^2$ für ein Wasserstoffatom steht und $R^3$ eine Methylgruppe darstellt,
$(R^1)_n$ eine 3-Pentyl- oder 4-Phenethylgruppe oder ein nicht-toxisches Salz desselben.

2. Verbindung nach Anspruch 1, worin bedeuten:
(i) $R^2$ eine Methylgruppe und $R^3$ ein Wasserstoffatom,
$(R^1)_n$ 3-Pentyl, 4-Pentyl, 2-Fluor-4-pentyloxy, 4-Cyclobutylmethyl, 4-Cyclohexylmethyl oder 4-Phenoxy und
(ii) wenn $R^2$ für ein Wasserstoffatom steht und $R^3$ eine Methylgruppe darstellt,
$(R^1)_n$ 3-Pentyl oder 4-Phenethyl.

3. Verbindung nach Anspruch 2, dadurch gekennzeichnet, daß $R^2$ für eine Methylgruppe steht, $R^3$ ein Wasserstoffatom bedeutet und $(R^1)_n$ 3-Pentyl, 4-Pentyl oder 4-Cyclobutylmethyl darstellt.

4. Verfahren zur Herstellung eines Cinnamoylamidderivats nach Anspruch 1, dadurch gekennzeichnet, daß man eine Verbindung der allgemeinen Formel:

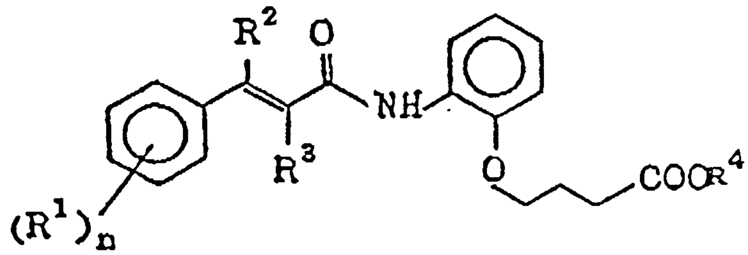

(II)

worin $R^4$ für eine gerad- oder verzweigtkettige Alkylgruppe mit 1 bis 4 Kohlenstoffatom(en) steht und die sonstigen Symbole die in Anspruch 1 angegebene Bedeutung besitzen, verseift und gegebenenfalls die erhaltene Verbindung der allgemeinen Formel (I) in ein nicht-toxisches Salz derselben umwandelt.

5. Verbindung der in Anspruch 4 angegebenen allgemeinen Formel (II), worin $R^4$ die in Anspruch 4 angegebene Bedeutung und die sonstigen Symbole die in Anspruch 1 angegebene Bedeutung besitzen.

6. Verfahren zur Herstellung einer Verbindung nach Anspruch 4, dadurch gekennzeichnet, daß man eine Verbindung der allgemeinen Formel:

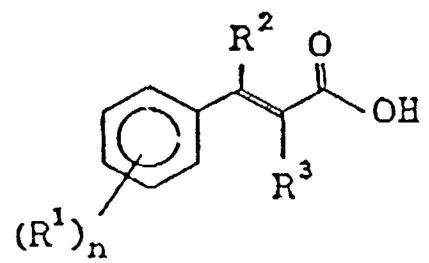

(III)

(worin sämtliche Symbole die in Anspruch 1 angegebene Bedeutung besitzen) oder ein aktiviertes Derivat derselben mit einem Amin der allgemeinen Formel:

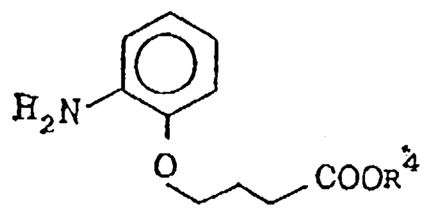

(IV)

worin $R^4$ die in Anspruch 4 angegebene Bedeutung besitzt, umsetzt.

**7.** Arzneimittel, enthaltend ein Cinnamoylamidderivat nach Anspruch 1 oder ein nicht-toxisches Salz desselben in Verbindung mit einem pharmazeutisch akzeptablen Träger oder Überzug.

**8.** Cinnamoylamidderivat nach Anspruch 1 oder ein nichttoxisches Salz desselben zur Verwendung als Arzneimittel.

**9.** Verwendung eines Cinnamoylamidderivats nach Anspruch 1 oder eines nicht-toxischen Salzes desselben bei der Herstellung eines Arzneimittels zur Behandlung von Alopezie, Akne oder Prostatavergrößerung.

**10.** Kosmetische Zubereitung, enthaltend ein Cinnamoylamidderivat nach Anspruch 1 oder ein nicht-toxisches Salz desselben.

**11.** Verwendung eines Cinnamoylamidderivats nach Anspruch 1 oder eines nicht-toxischen Salzes desselben als kosmetisches Produkt bei der Behandlung von Akne oder Alopezie.

**Patentansprüche für folgende Vertragsstaaten : ES, GR**

**1.** Verfahren zur Herstellung eines Cinnamoylamidderivats der allgemeinen Formel:

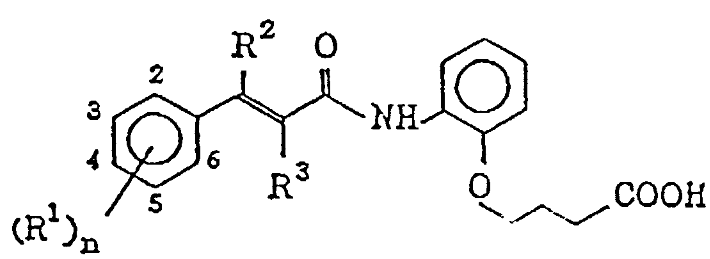

(I)

worin bedeuten:

(i) wenn $R^2$ für eine Methylgruppe steht und $R^3$ ein Wasserstoffatom darstellt,

$(R^1)_n$ 3-Pentyl, 4-Pentyl, 4-Neopentyl, 4-(2-Ethylbutyl), 4-(2-Methylpentyl), 2-Fluor-4-pentyloxy, 4-Butylthio, 4-Cyclobutylmethyl, 4-Cyclohexylmethyl, 4-(4-Phenylbutyl) oder 4-Phenoxy und
(ii) wenn $R^2$ für ein Wasserstoffatom steht und $R^3$ eine Methylgruppe darstellt,
$(R^1)_n$ eine 3-Pentyl- oder 4-Phenethylgruppe oder eines nicht-toxischen Salzes desselben, dadurch gekennzeichnet, daß man eine Verbindung der allgemeinen Formel:

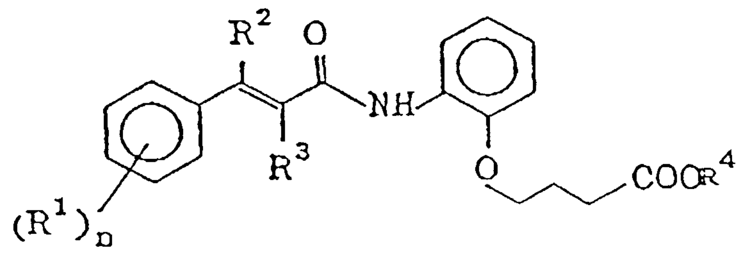

(II)

worin $R^4$ für eine gerad- oder verzweigtkettige Alkylgruppe mit 1 bis 4 Kohlenstoffatom(en) steht und die sonstigen Symbole die bezüglich der allgemeinen Formel (I) angegebene Bedeutung besitzen, verseift und gegebenenfalls die erhaltene Verbindung der allgemeinen Formel (I) in ein nicht-toxisches Salz derselben umwandelt.

**2.** Verfahren nach Anspruch 1 zur Herstellung einer Verbindung der Formel (I) oder eines nicht-toxischen Salzes derselben, worin bedeuten:
(i) $R^2$ eine Methylgruppe und $R^3$ ein Wasserstoffatom,
$(R^1)_n$ 3-Pentyl, 4-Pentyl, 2-Fluor-4-pentyloxy, 4-Cyclobutylmethyl, 4-Cyclohexylmethyl oder 4-phenoxy und
(ii) wenn $R^2$ für ein Wasserstoffatom steht und $R^3$ eine Methylgruppe darstellt,
$(R^1)_n$ 3-Pentyl oder 4-Phenethyl.

**3.** Verfahren nach Anspruch 2 zur Herstellung einer Verbindung der Formel (I) oder eines nicht-toxischen Salzes derselben, worin $R^2$ für eine Methylgruppe steht, $R^3$ ein Wasserstoffatom bedeutet und $(R^1)_n$ 3-Pentyl, 4-Pentyl oder 4-Cyclobutylmethyl darstellt.

**4.** Verfahren zur Herstellung einer Verbindung der in Anspruch 1 dargestellten allgemeinen Formel (II), dadurch gekennzeichnet, daß man eine Verbindung der allgemeinen Formel:

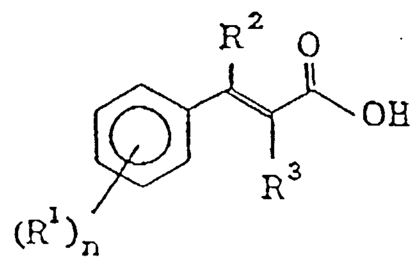

(III)

(worin sämtliche Symbole die in Anspruch 1 angegebene Bedeutung besitzen) oder ein aktiviertes Derivat derselben mit einem Amin der allgemeinen Formel:

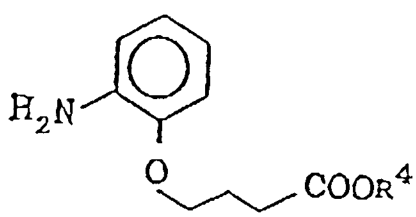

(IV)

worin $R^4$ die in Anspruch 1 angegebene Bedeutung besitzt, umsetzt.

5. Kosmetische Zubereitung, enthaltend ein Cinnamoylamidderivat der in Anspruch 1 dargestellten Formel (I) oder ein nicht-toxisches Salz desselben.

**Revendications**

**Revendications pour les Etats contractants suivants : AT, BE, CH, DE, FR, GB, IT, LI, LU, NL, SE**

1. Dérivé d'amide cinnamique, ou de cinnamoylamide, de formule générale :

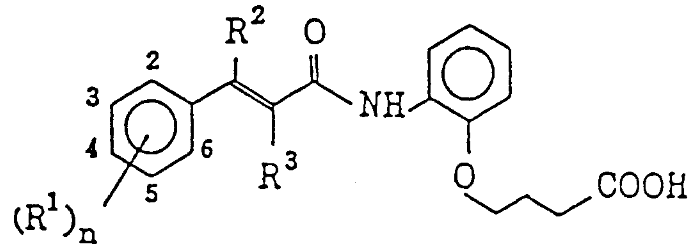

(I)

[dans laquelle
(i) lorsque $R^2$ représente un groupe méthyle, et que $R^3$ représente un atome d'hydrogène,
$(R^1)_n$ représente un groupe 3-pentyle, 4-pentyle, 4-néopentyle, 4-(2-éthylbutyle), 4-(2-méthylpentyle), 2-fluoro-4-pentyloxy, 4-butylthio, 4-cyclobutylméthyle, 4-cyclohexylméthyle, 4-(4-phénylbutyle) ou 4-phénoxy, et
(ii) lorsque $R^2$ représente un atome d'hydrogène et que $R^3$ représente un groupe méthyle,
$(R^1)_n$ représente un groupe 3-pentyle ou 4-phénéthyle] ou un sel non toxique de ce dérivé.

2. Composé selon la revendication 1, dans lequel
(i) $R^2$ représente un groupe méthyle et $R^3$ représente un atome d'hydrogène,
$(R^1)_n$ représente un groupe 3-pentyle, 4-pentyle, 2-fluoro-4-pentyloxy, 4-cyclobutylméthyle, 4-cyclohexylméthyle ou 4-phénoxy, et
(ii) lorsque $R^2$ représente un atome d'hydrogène et que $R^3$ représente un groupe méthyle,
$(R^1)_n$ représente un groupe 3-pentyle ou 4-phénéthyle.

3. Composé selon la revendication 2, dans lequel $R^2$ représente un groupe méthyle, $R^3$ représente un atome d'hydrogène et $(R^1)_n$ représente un groupe 3-pentyle, 4-pentyle ou 4-cyclobutylméthyle.

4. Procédé pour la préparation d'un dérivé d'amide cinnamique selon la revendication 1, qui comprend la saponification d'un composé de formule générale :

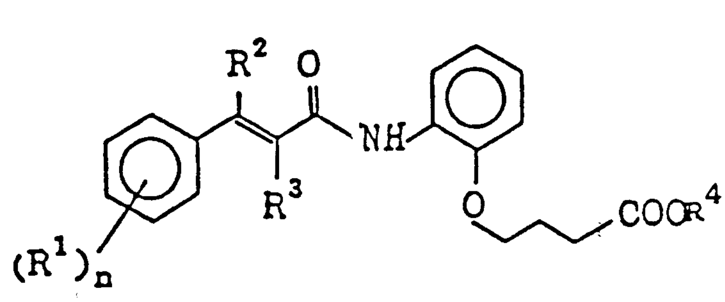

(II)

dans laquelle $R^4$ représente un groupe alkyle linéaire ou ramifié ayant 1 à 4 atomes de carbone, et les autres symboles sont tels que définis à la revendication 1, et éventuellement la transformation du composé de formule générale (I), ainsi obtenu, en un sel non toxique de ce composé.

5. Composé de formule générale (II) définie à la revendication 4, dans lequel $R^4$ est tel que défini à la revendication 4 et les autres symboles sont tels que définis à la revendication 1.

**6.** Procédé pour la préparation d'un composé selon la revendication 4, qui comprend la réaction d'un composé de formule générale :

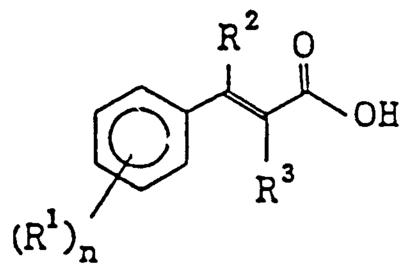

(III)

dans laquelle tous les symboles sont tels que définis à la revendication 1 ou d'un dérivé activé de ce composé, avec une amine de formule générale :

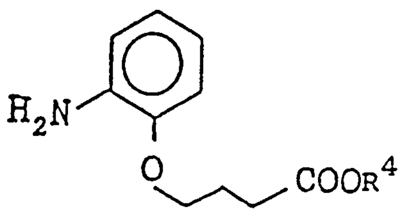

(IV)

dans laquelle $R^4$ est tel que défini à la revendication 4.

**7.** Composition pharmaceutique, qui comprend un dérivé d'amide cinnamique selon la revendication 1 ou un sel non toxique de ce dérivé, en association avec un véhicule, excipient ou revêtement pharmaceutiquement acceptables.

**8.** Dérivé d'amide cinnamique selon la revendication 1, ou son sel non toxique, destiné à servir de médicament.

**9.** Utilisation du dérivé d'amide cinnamique selon la revendication 1, ou d'un sel non toxique de ce dérivé, dans la fabrication d'un médicament pour le traitement de l'alopécie, de l'acné ou de l'hypertrophie de prostate.

**10.** Composition cosmétique, qui comprend un dérivé d'amide cinnamique selon la revendication 1, ou un sel non toxique de ce dérivé.

**11.** Utilisation, comme produit cosmétique dans le traitement de l'acné ou de l'alopécie, d'un dérivé d'amide cinnamique selon la revendication 1 ou d'un sel non toxique de ce dérivé.

**Revendications pour les Etats contractants suivants : ES, GR**

**1.** Procédé pour la préparation d'un dérivé d'amide cinnamique (ou de cinnamoylamide) de formule générale :

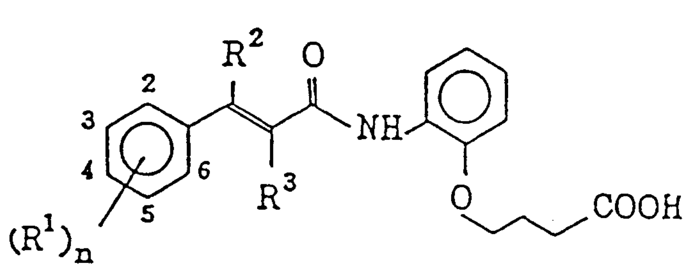

(I)

[dans laquelle

(i) lorsque $R^2$ représente un groupe méthyle et que $R^3$ représente un atome d'hydrogène,
$(R^1)_n$ représente un groupe 3-pentyle, 4-pentyle, 4-néopentyle, 4-(2-éthylbutyle), 4-(2-méthylpentyle), 2-fluoro-4-pentyloxy, 4-butylthio, 4-cyclobutylméthyle, 4-cyclohexylméthyle, 4-(4-phénylbutyle) ou 4-phénoxy, et

(ii) lorsque $R^2$ représente un atome d'hydrogène et que $R^3$ représente un groupe méthyle,
$(R^1)_n$ représente un groupe 3-pentyle ou 4-phénéthyle] ou un sel non toxique de ce composé, ce procédé comprenant la saponification d'un composé de formule générale :

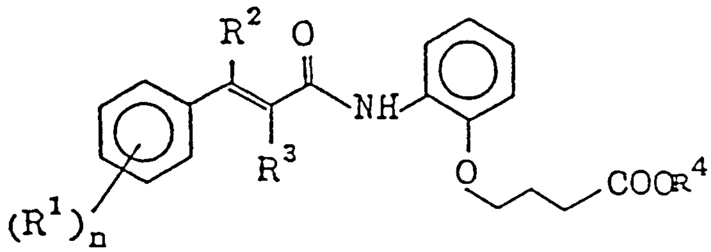

(II)

dans laquelle $R^4$ représente un groupe alkyle, linéaire ou ramifié, ayant 1 à 4 atomes de carbone, et les autres symboles sont tels que définis à propos de la formule générale (I), et éventuellement la transformation du composé de formule générale (I), ainsi obtenu, en un sel non toxique de ce composé.

**2.** Procédé selon la revendication 1, pour la préparation d'un composé de formule (I) ou d'un sel non toxique de ce composé, dans lequel (i) $R^2$ représente un groupe méthyle et $R^3$ représente un atome d'hydrogène,
$(R^1)_n$ représente un groupe 3-pentyle, 4-pentyle, 2-fluoro-4-pentyloxy, 4-cyclobutylméthyle, 4-cyclohexylméthyle ou 4-phénoxy, et (ii) lorsque $R^2$ représente un atome d'hydrogène et que $R^3$ représente un groupe méthyle,
$(R^1)_n$ représente un groupe 3-pentyle ou 4-phénéthyle.

**3.** Procédé selon la revendication 2, pour la préparation d'un composé de formule (I) ou d'un sel non toxique de ce composé, dans lequel $R^2$ représente un groupe méthyle, $R^3$ représente un atome d'hydrogène et $(R^1)_n$ représente un groupe 3-pentyle, 4-pentyle ou 4-cyclobutylméthyle.

**4.** Procédé pour la préparation d'un composé de formule générale (II), définie à la revendication 1, ce procédé comprenant la réaction d'un composé de formule générale :

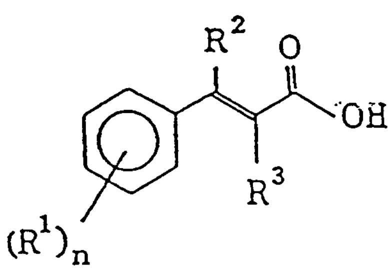

(III)

dans laquelle tous les symboles sont tels que définis à la revendication 1, ou d'un dérivé activé de ce composé, avec une amine de formule générale :

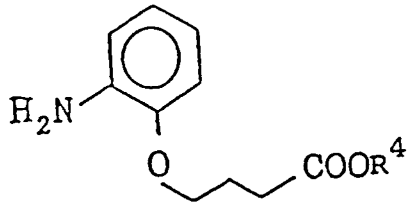

dans laquelle $R^4$ est tel que défini à la revendication 1.

5. Composition cosmétique, qui comprend un dérivé d'amide cinnamique de formule (I), tel que défini à la revendication 1, ou un sel non toxique de ce dérivé.